# EUROPEAN PATENT SPECIFICATION

(11) **EP 3 458 717 B1**
(45) Date of publication and mention of the grant of the patent: **16.06.2021**
(21) Application number: 17726819.0
(22) Date of filing: 19.05.2017
(51) Int. Cl.: F04B 43/12

(54) **PUMP**
PUMPE
POMPE

(30) Priority: 20.05.2016 US 201662339666 P
(43) Date of publication of application: 27.03.2019
(73) Proprietor: Terumo BCT, Inc., Lakewood, CO 80215 (US)
(72) Inventor: O'BRIEN, Peter, Denver Colorado 80210 (US); GOBLIRSCH, John, Denver Colorado 80210 (US)
(74) Representative: J A Kemp LLP
(86) International application number: PCT/US2017/033587
(87) International publication number: WO 2017/201434

(56) References cited:
- WO-A1-95/17597
- WO-A1-99/42726
- US-A- 5 263 831
- US-A1- 2016 131 127

## Description

### Background

Various types of pumps are used in medical devices for pumping liquids to and from various systems, patients, and/or devices. Frequently, the pumps are peristaltic pumps that are used to move fluid within tubing that may be sterile. The pumps therefore may engage with the tubing, compressing the tubing to move fluid.

Embodiments have been made in light of these and other considerations. However, the relatively specific problems discussed above do not limit the applicability of the embodiments of the present disclosure.

US 5263831 A discloses a peristaltic pump.

### Summary

The summary is provided to introduce aspects of some embodiments in a simplified form, and is not intended to identify key or essential elements, nor is it intended to limit the scope of the claims.

Embodiments are described that provide for a pump that includes a first portion and a second portion. The first portion includes at least one roller for engaging tubing. The second portion includes a raceway that may be opposed to the at least one roller so that when the roller engages the tubing, the tubing is pressed against the raceway by the roller. The pump further comprises a track, at least a portion of the track coming in contact with the roller when the roller is not engaged with the tubing. In embodiments, the roller may include a top edge or a bottom edge that is rounded.

In other embodiments, the first portion comprises an indicator pin that indicates a loading state of the pump. The indicator pin may have different heights depending on the loading state of the pump.

### Brief Description of the Drawings

Non-limiting and non-exhaustive embodiments are described with reference to the following figures.
FIG. 1 illustrates a separation system that may be used to separate a multi-component fluid (e.g., whole blood) into components consistent with an embodiment.
FIG. 2 illustrates a tubing and bag set that may be used in a separator for separating components of a multi-component fluid (e.g., whole blood) consistent with an embodiment.
FIG. 3 depicts a perspective view of a pump, in accordance with embodiments.
FIG. 4 depicts a perspective view of a first portion of the pump shown in FIG. 3, in accordance with embodiments.
FIG. 5 depicts a front view of the first portion shown in FIG. 4, in accordance with embodiments.
FIG. 6 depicts a side view of the first portion shown in FIG. 4, in accordance with embodiments.
FIG. 7 depicts a first cross-sectional view of the first portion shown in FIG. 4 (taken along line AA), in accordance with embodiments.
FIG. 8 depicts a second cross-sectional view of the first portion shown in FIG. 4 (taken along line BB), in accordance with embodiments.
FIG. 9 depicts a perspective view of a second portion of the pump shown in FIG. 3, in accordance with embodiments.
FIG. 10 depicts a cross-sectional view of the pump of FIG. 3 (taken along line CC), in accordance with embodiments.
FIG. 11 depicts a cross-sectional view of the pump of FIG. 3 (taken along line CC), in accordance with embodiments.
FIG. 12 illustrates another view of the second portion of the pump of FIG. 3.
FIG. 13 illustrates a front view of a roller, in accordance with embodiments.
FIG. 14 illustrates a perspective view of a first portion of a pump according to other embodiments.
FIG. 15 illustrates a perspective view of a first portion of yet another embodiment of a pump.
FIGS. 16A-C illustrates a top view of another embodiment of a pump with an indicator pin at different positions (e.g., heights relative to a cap).
FIG. 17 illustrates a flow chart of a process for operating a pump.

### Detailed Description

The principles of the present disclosure may be further understood by reference to the following detailed description and the embodiments depicted in the accompanying drawings. It should be understood that although specific features are shown and described below with respect to detailed embodiments, the present disclosure is not limited to the embodiments described below. Also, the embodiments may be described in relation to use in a blood separation system. It is noted that this is merely for illustrative purposes. The embodiments may be used in any system that provides for moving of fluids through tubing or other conduits.

Reference will now be made in detail to the embodiments illustrated in the accompanying drawings and described below. Wherever possible, the same reference numerals are used in the drawings and the description to refer to the same or like parts.

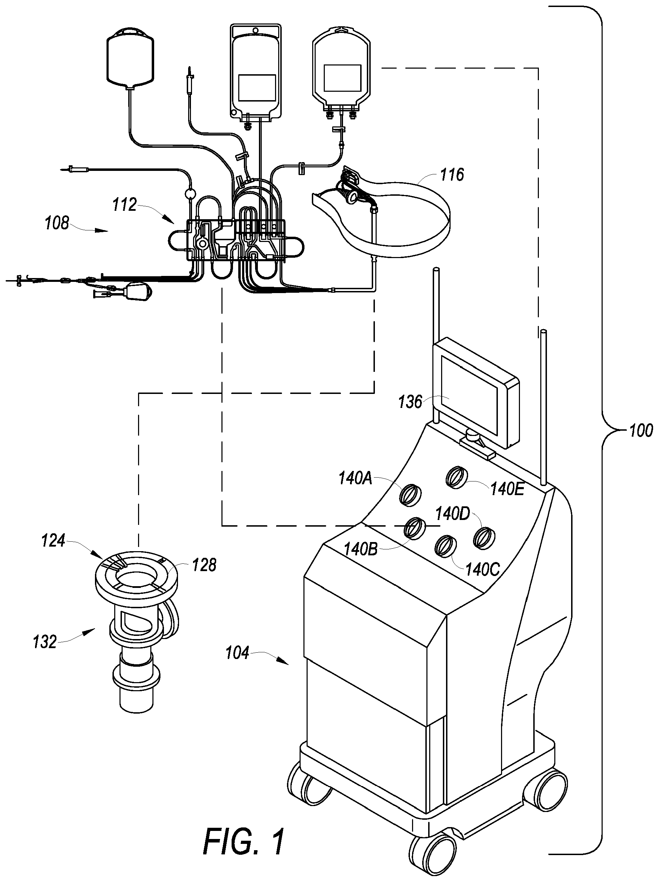

FIG. 1 illustrates one embodiment of a separation system 100, which can be used in, or with, embodiments. In some embodiments, separation system 100 provides for a continuous whole blood separation process. In one embodiment, whole blood is withdrawn from a donor and is substantially, continuously provided to a separation device 104 where the blood may be separated into various components and at least one of these components may be collected. One or more of the separated components may be either collected for subsequent use or returned to the donor. In embodiments, blood may be withdrawn from the donor and directed through a bag and tubing set 108, which includes a tubing circuit 112, and a fluid processing vessel 116, which together define a closed, sterile and disposable system. The set 108 may be adapted to be mounted in the separation device 104. The separation device 104 includes pumps 140A-E, which interface with the tubing circuit 112; and a centrifuge assembly 124, which interfaces with the fluid processing vessel 116.

Non-limiting examples of separation systems that may be the basis of systems used with embodiments of the present invention, e.g., separation system 100, include the SPECTRA OPTIA® apheresis system, COBE® spectra apheresis system, and the TRIMA ACCEL® automated blood collection system, all manufactured by Terumo BCT, Inc. of Lakewood, Colorado.

The centrifuge assembly 124 may include a channel 128 in a rotatable rotor assembly 132 (e.g., centrifuge), where the channel 128 may be used to hold a fluid processing vessel, e.g., vessel 116. The rotor assembly 132 may rotate to create a centrifugal field. The rotor assembly 132 may be configured to hold a chamber used to separate, concentrate, and/or wash cells. In one example, when whole blood is processed, cellular components of blood may be separated from each other and from liquid components of blood.

The fluid processing vessel 116 may be fitted within the channel 128. In one example, blood can flow substantially continuously from a donor, through the tubing circuit 112, and into the rotating fluid processing vessel 116. Within the fluid processing vessel 116, blood may be separated into various blood component types and at least one of these blood component types (e.g., white blood cells, platelets, plasma, red blood cells, or combinations thereof) may be removed from the fluid processing vessel 116 and further processed. Blood components that are not being retained for collection or for therapeutic treatment (e.g., red blood cells, platelets, white blood cells, and/or plasma) may also be removed from the fluid processing vessel 116 and returned to the donor via the tubing circuit 112.

Operation of the separation device 104 may be controlled by one or more processors included therein, and may comprise a plurality of embedded computer processors that are part of a computer system. The computer system may include a number of components, such as, memory and storage devices (RAM, ROM (e.g., CD-ROM, DVD), magnetic drives, optical drives, flash memory,); communication/networking devices (e.g., wired such as modems/network cards, or wireless such as Wi-Fi); input devices such keyboard(s), touch screen(s), camera(s), and/or microphone(s); and output device(s) such as display(s), and audio system(s). The computer system may, in embodiments, control one or more pumps, valves, sensors, etc. In order to interface with an operator of the system 100, embodiments of the separation device 104 may include a graphical user interface 136 (shown in FIG. 1) with a display that includes an interactive touch screen.

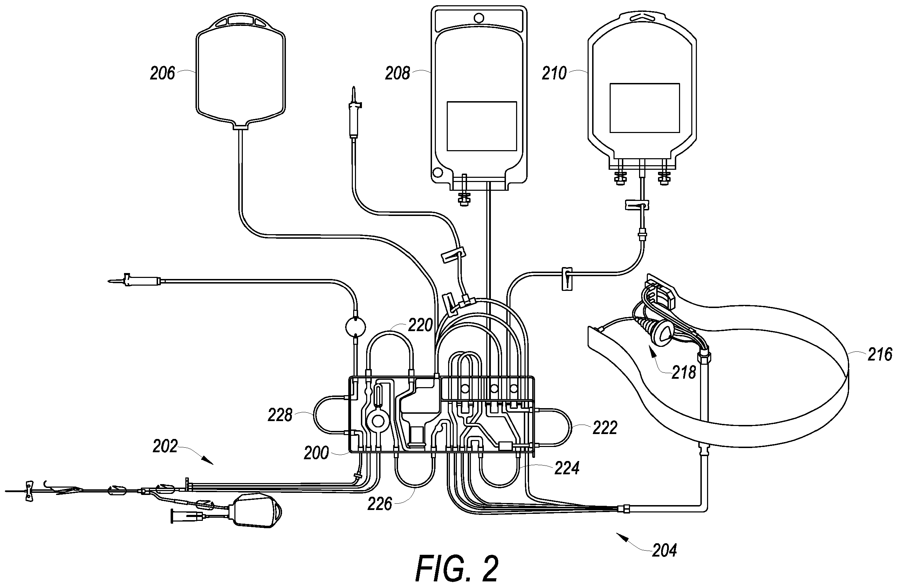

Pumps 140A-E may be used to pump liquid through tubing to move liquid to various parts of system 100 and/or donors/patients. An example of a tubing set with tubing loops that may loaded into, and engaged by, portion of pumps 140A-E is described below with respect to FIG. 2. Some embodiments of pumps 140A-E, and some of their features, are described below and depicted in the figures.

An embodiment of a tubing set (e.g., tubing set 108) that may be used with embodiments is shown in FIG. 2. The tubing set may include a cassette 200 and a number of tubing/storage assemblies 202, 204, 206, 208, and 210. In addition, tubing loops 220, 222, 224, 226, and 228 may engage with peristaltic pumps on a separation device, e.g., device 104 and pumps 140A-E, to pump fluids through the tubing/storage assemblies. The tubing set may also include vessel 216 and chamber 218.

In embodiments, the tubing set shown in FIG. 2 may be used to separate whole blood into components. In embodiments, some components separated from whole blood may be returned to a donor, stored in one or more storage containers, or further processed. For example, whole blood may be circulated through tubing of the tubing set and into the fluid processing vessel 216, which is mounted on a rotor assembly (e.g., assembly 128). Chamber 218 may also be mounted on the rotor assembly.

In the fluid processing vessel 216, the blood may separate into components. Some components may be returned to a donor while others may be further processed. For example, chamber 218 may be used to further process (concentrate or separate) components. In one embodiment, platelets, plasma, and white blood cells may be directed to chamber 218 where they may be further processed (concentrated, separated, etc.) before being stored in a container (e.g., a bag) or returned to a donor. In some of these embodiments, chamber 218 may be designed to concentrate platelets and generate a platelet product with as few white blood cells as possible. In other embodiments, red blood cells separated from whole blood may be introduced into chamber 218 and concentrated before being stored in a container (e.g., a bag). These are merely some examples and embodiments may separate and concentrate other components of whole blood, or other composite fluid.

Some examples of separation systems (that may be used in embodiments) that includes a blood processing vessel and tubing sets connecting various portions of separation systems are described in U.S. Patent No. 6,902,539, entitled "EXTRACORPOREAL BLOOD PROCESSING METHODS AND APPARATUS" issued, June 7, 2005; U.S. Patent No. 6,613,009, entitled "EXTRACORPOREAL BLOOD PROCESSING METHODS AND APPARATUS" issued, September 2, 2003; U.S. Patent No. 5,702,357, entitled "EXTRACORPOREAL BLOOD PROCESSING METHODS AND APPARATUS" issued, December 30, 1997.

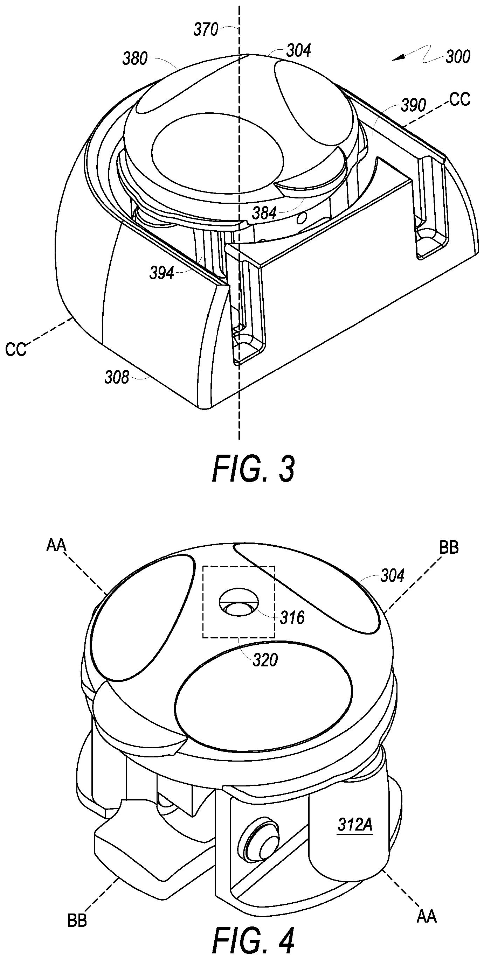

FIGS. 3-13 depict various portions of an embodiment of a pump 300 (e.g., a peristaltic pump) that may be implemented in some embodiments. For example, pumps 140-A-E may be implemented using embodiments of pump 300. In some embodiments, pump 300 includes features that allow quiet operation of the pump 300. By "quiet" it is meant, a reduction in noise and/or vibration relative to a pump that does not include the features being described.

As shown in FIG. 3, pump 300 includes a first portion 304 and a second portion 308. As may be appreciated, in embodiments, first portion 304 is configured to rotate, while it is connected to second portion 308. In embodiments, first portion rotates around an axis of rotation 370. At least some of first portion 304 is within second portion 308 when portion 304 is rotated around axis of rotation 370. A tubing loop (e.g., 220, 222, 224, 226, and 228) may be designed to fit between the first portion 304 and the second portion 308 by being routed around first portion 304.

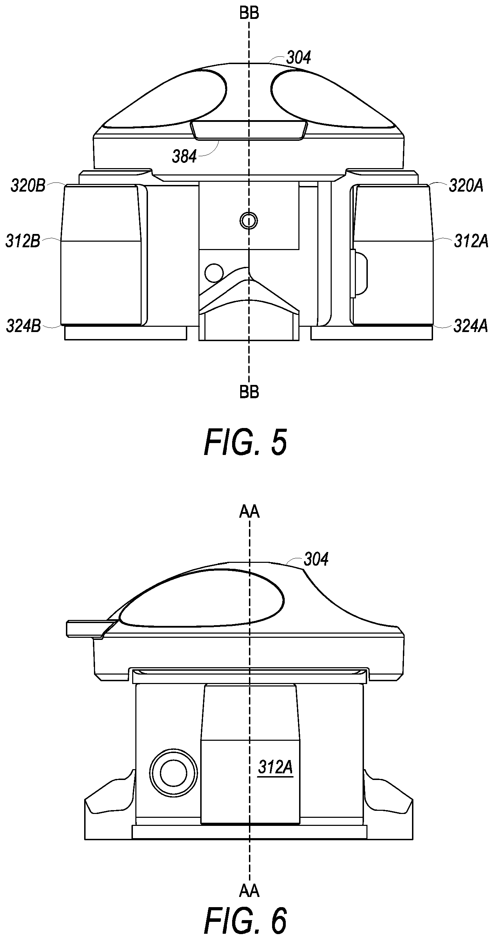

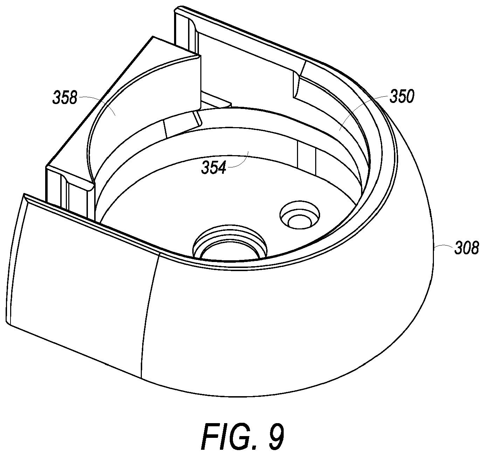

As shown in FIGS. 4-8, first portion 304 includes roller 312A, roller 312B, indicator 316, and area 320 adjacent indicator 316. Rollers 312A and 312B are generally cylindrical in nature (FIGS. 5-7). Roller 312A includes a top edge 320A and a bottom edge 324A. In embodiments, one or more of top edge 320A and/or 320B may have a rounded edge. Similarly, roller 312B includes a top edge 320B and a bottom edge 324B. In embodiments, one or more of top edges 320A/320B and/or bottom edges 324A/324B may have a rounded edge. The rounded edge may help in engaging tubing. Also, in some embodiments, the rounded edge may create less noise when roller 312A/312B is in contact with raceway 350, or track 354, of second portion 308 (FIG. 9).

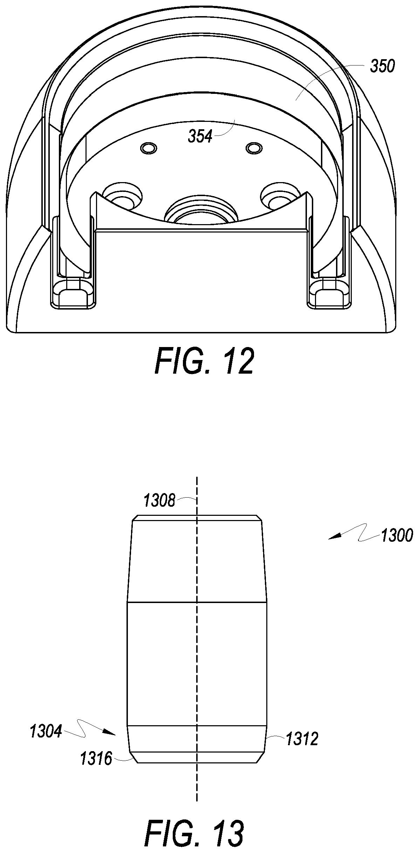

FIG. 13 shows a front view of one embodiment of a roller 1300 with a rounded edge 1304. Rounded edge 1304 includes a first portion 1312 which is slanted inward toward a center line 1308. The second portion 1316 is slanted further inward toward centerline 1308. The rounded edge 1304 may in embodiments further reduce noise associated with operation of a pump when roller 1300 is in contact with a track (e.g. track 354) and/or a raceway (e.g. raceway 350). Roller 1300 may be used as rollers 312A and/or 312B in some embodiments.

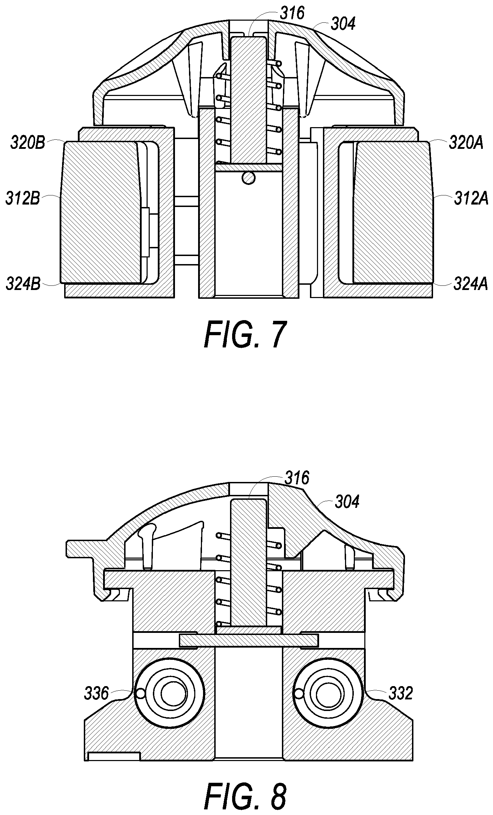

Referring now to FIG. 8, each of rollers 312A and 312B may be spring loaded by for example springs 332 and/or 336 (FIG. 8). Springs 332 and/or 336 may push rollers radially away from axis of rotation 370. Thus, rollers 312A and 312B have some range of motion, e.g., springs 332 and/or 336 may be compressed such as when tubing is in channel 352.

FIGS. 9 and 12 depict perspective views of second portion 308. Second portion 308 includes a raceway 350, a track 354, and curved portion 358. When first portion 304 is connected to second portion 308 and first portion is rotated around axis of rotation 370, at least a portion of rollers 312A and 312B may come in contact with track 354. As described in greater detail below, the contact of rollers 312A and 312B with track 354 allows for a quiet operation of pump 300.

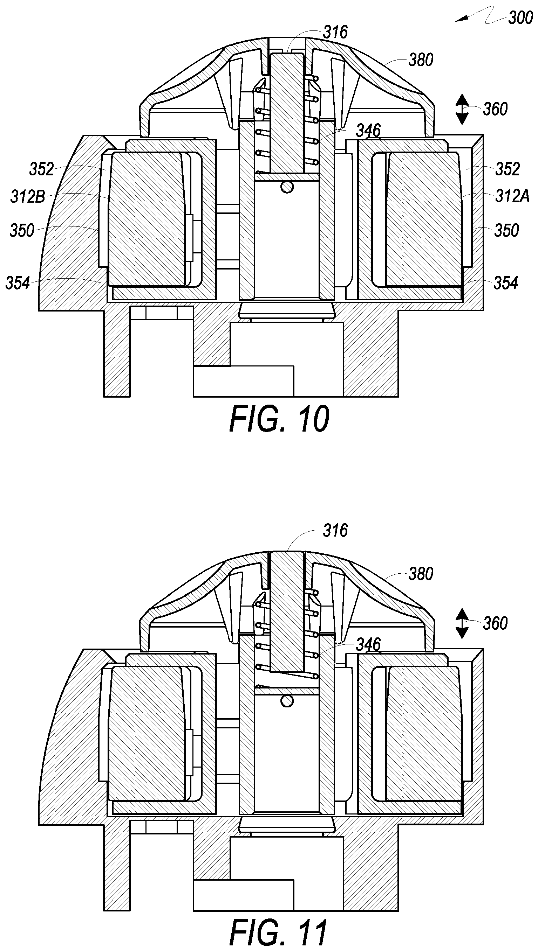

FIG. 10 illustrates a cross-sectional view of pump 300 taken along line CC (FIG. 3). As shown in FIG. 10, when portion 304 is connected to portion 308, a space exists between rollers 312A/312B and raceway 350, e.g., channel 352. When pump 300 is in operation, tubing, e.g., a tubing loop (e.g., 220, 222, 224, 226, and 228) is positioned within this space (e.g., channel 352). The rotation of portion 304 causes the rollers to squeeze the tubing and move fluid in the tubing. As also shown in FIG. 10, track 354 remains in contact with the rollers, even if there is no tubing in the pump, i.e., the space between raceway 350 and the rollers (e.g., channel 352).

The fact that the rollers 312A/313B remain in contact with the track 354 may result in quiet operation of the pump 300. Without track 354, rollers (because of their spring loading) may move (be pushed radially out by the springs 332/336) when tubing is not positioned in the space (e.g., channel 352) between raceway 350 and the rollers 312A/312B. This may create vibration and/or noise. Accordingly, track 354 may provide for quiet operation of pump 300. Additionally, curved portion 358 may also include a portion of track 354 that also maintains the position of the rollers 312A/312B even if not in contact with tubing. In other embodiments, track 354 may be separate from curved portion 358.

As may be appreciated, in embodiments, rollers 312A/312B may be engaged with tubing at different times. That is, as first portion 304 rotates, roller 312A may be engaged with tubing, while roller 312B may not. As first portion continues to rotate, roller 312A may disengage with the tubing while roller 312B engages the tubing.

For example, in a first transition zone 390, the roller (312A/312B) may engage the tubing and not be in contact with the track 354. In other words, tubing in channel 352 may push against the roller (312A/312B) and cause it to lose contact with the track 354. As the roller (312A/312B) rotates around the raceway 350, in a second transition zone 394, the roller (312A/312B) may disengage the tubing and contact the track 354, which limits the radial movement of the roller (312A/312B).

In some embodiments, the track 354 may be a loop that extends around the entire path travelled by the rollers 312A/312B including the length of the raceway. In embodiments, the track 354 may at some locations be flush with at least a portion of the raceway 350. In other embodiments, the track 354 may be a partial loop and extend around only a portion of the path travelled by the rollers 312A/312B. In these embodiments, the track 354 may extend around only a portion of the length of the raceway 350. For example, the track 354 may, in embodiments, only extend into the transition zones 390 and 394 but not fully around the entire path travelled by the rollers 312A/312B.

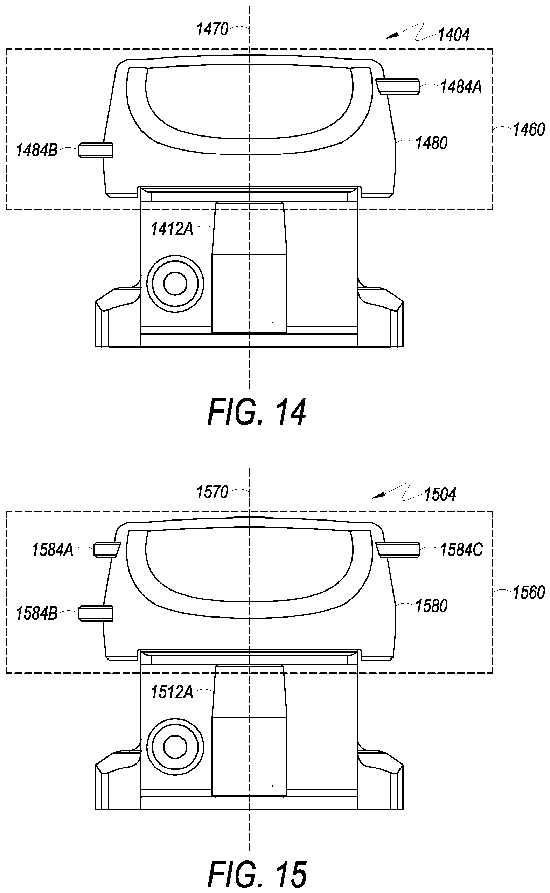

Pump 300 also includes tab 384 on cap 380 (FIG. 8). In embodiments, tab 384 may be used to automatically load a tubing loop (e.g., loops 220, 222, 224, 226, and 228). As first portion 304 rotates around axis of rotation 370 tab 384 may catch the tubing loop and guide the tubing loop toward the second portion 308, rollers 312A/312B, and into the channel 352. Additional tab designs and arrangements that may be included in some embodiments are described below with respect to FIGS. 14 and 15.

FIGS. 10 and 11 also illustrate a feature of some embodiments. Indicator 316 (which is part of first portion 304) is spring loaded using spring 346. FIG. 10 illustrates indicator 316 when first position 304 is in an open state. In embodiments, a cap 380 of first portion 304 may be movable as shown by arrow 360. Cap 380 may be moved upward (open position) to allow tubing to be fit around top portion 304 in channel 352.

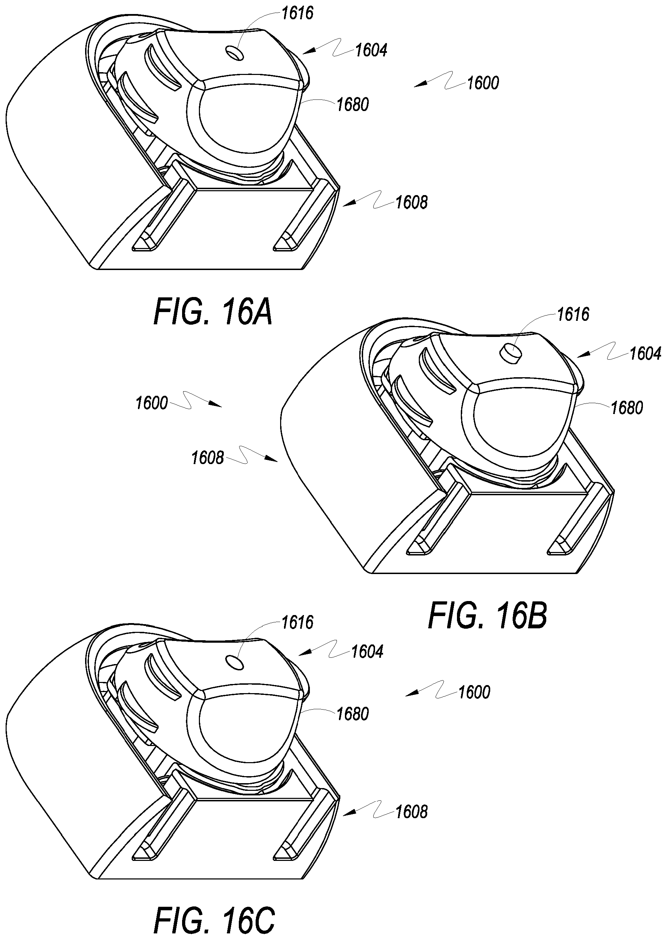

FIG. 11 illustrates indicator 316 when first position 304 is in a closed state. In embodiments, cap 380 of first portion 304 may be movable as shown by arrow 360. Cap 380 may be moved downward (closed position) to secure tubing and move the tubing into the space (e.g., channel 352) between raceway 350 and the rollers. As shown in FIG. 10, indicator 316 is popped up to indicate that first portion is in the closed position. In some embodiments, the top of indicator 316 may have a color that is different from an area 320 (FIG. 3) of the first portion 304 adjacent the indicator 316. For example, area 320 may be a first color and the top of indicator 316 may be a different color (e.g., yellow, green, blue, red, or white etc.) so that an operator can easily determine the loading state of pump 300. Alternative indicator features that may be included in some embodiments are described below with respect to FIGS. 16A-C.

FIG. 14 illustrates another embodiment of a first portion 1404 of a pump. As shown in FIG. 14, first portion 1404 includes features that may be similar to some features described above with respect to first portion 304. In embodiments, first portion 1404 may fit within a second portion (e.g., second portion 308) and rotate around axis of rotation 1470 to engage tubing and move fluid through the tubing. For example, first portion 1404 may include rollers 1412A and 1412B (not shown). In embodiments, first portion 1404 may be used as first portion 304 in pump 300 described above.

In the embodiment shown in FIG. 14, first portion 1408 includes a cap 1480 with a plurality of tabs, namely tabs 1484A and 1484B. As shown in FIG. 14, tabs 1484A and 1484B extend radially away from axis of rotation 1470. Furthermore, tabs 1484A and 1484B are diametric to each other. By "diametric" it is meant that tab 1484A and 1484B are on opposite sides of first portion 1404, such that, if first portion 1404 is sectioned along a diameter, e.g., plane 1460, the section would pass through at least a portion of both tabs 1484A and 1484B. In addition, tab 1484B is also located lower, e.g., closer to where a second portion would be.

In operation, first portion 1404 would be within a second portion, e.g., second portion 308, as part of a pump. A tubing loop, e.g., tubing loop 220, 222, 224, 226, and 228, may be placed around first portion 1404. As first portion 1404 rotates around axis of rotation 1470, the tubing loop may be guided downward along cap 1480 by tabs 1484A and 1484B and toward a second portion which may form a channel (e.g., channel 352) with first portion 1404, for example with roller 1412A. The tubing loop may first be caught on a bottom of tab 1484A, which would guide the tubing loop downward so that tab 1484B may catch the tubing loop and guide it even further down along cap 1480. In this way, tabs 1484A and 1484B may aid in automatically loading a tubing loop into a pump channel.

In embodiments, tabs 1484A and 1484B may have different sizes. For example, in the embodiment shown in FIG. 14 tab 1484A extends further out from cap 1480 than tab 1484B. In embodiments, this may aid in moving the tubing loop downward toward the second portion of the pump, since the smaller size of tab 1484B may allow the tubing loop to more easily slip below tab 1484B (and be guided lower toward the channel) than if tab 1484B extended further.

FIG. 15 illustrates yet another embodiment of a first portion 1504 of a pump. As shown in FIG. 15, first portion 1504 includes features that may be similar to some features described above with respect to first portion 304. In embodiments, first portion 1504 may fit within a second portion (e.g., second portion 308) and rotate around axis of rotation 1570 to engage tubing and move fluid through the tubing. For example, first portion 1504 may include rollers 1512A and 1512B (not shown). In embodiments, first portion 1504 may be used as first portion 304 in pump 300 described above.

In the embodiment shown in FIG. 15, first portion 1508 includes a cap 1580 with a plurality of tabs, namely tabs 1584A, 1584B, and 1584C. As shown in FIG. 15, tabs 1584A, 1584B, and 1584C extend radially away from axis of rotation 1570. Furthermore, tabs 1584A and 1584C are diametric to each other. As noted above, by "diametric" it is meant that tabs 1584A and 1584C are on opposite sides of first portion 1504, such that, if first portion 1504 is sectioned along a diameter, e.g., plane 1560, the section would pass through at least a portion of both tabs 1584A and 1584C. In addition, tab 1584B is located adjacent 1584A, however it is lower on cap 1580, e.g., closer to where a second portion would be. Tab 1584B and 1584C are also diametric.

In operation, first portion 1504 would be within a second portion, e.g., second portion 308, as part of a pump. A tubing loop, e.g., tubing loop 220, 222, 224, 226, and 228, may be placed around first portion 1504. As first portion 1504 rotates around axis of rotation 1570, the tubing loop may be caught by tabs 1584A and 1584C and be guided downward along cap 1580 toward a second portion of the pump which may form a channel (e.g., channel 352) with first portion 1504. As the tubing loop moves down cap 1580, tab 1584B may catch the loop and guide it further down toward the second portion. In this way, tabs 1584A, 1584B, and 1584C may aid in automatically loading a tubing loop into a pump.

The description above of different tab designs, shapes, layouts, positions, etc. shown in FIGS. 14 and 15 is provided for illustrative purposes only. In other embodiments, a first portion may include more than two or three tabs, some of which have different shapes, sizes, and positions. For example, referring to first portion 1508, some embodiments may in addition to including tabs 1584A, 1584B, and 1584C, may include an additional tab adjacent 1584C and lower along cap 1580, similar to tab 1584B. This is merely one additional example, and other designs are within the scope of the present invention.

FIGS. 16A-C illustrate a pump 1600 in various states. Pump 1600 includes a first portion 1604 and a second portion 1608. In operation, first portion rotates around an axis of rotation within second portion 1608. First portion 1604 includes a cap 1680 and an indicator pin 1616. Indicator pin 1616 may operate, similar to indicator 316 described above (FIG. 3). In the embodiments shown in FIG. 16A-C, the height of indicator pin 1616 (in relation to a top of cap 1680) provides an indication as to the loading state of pump 1600.

In embodiments, when the pump 1600 is not loaded with a tubing loop, indicator pin 1616 may be positioned to a height bellow a top of cap 1680 as shown in FIG. 16A. That is, indicator 1616 will be at a height that positions it substantially within cap 1680. This may provide a visual indication to an operator that the pump 1600 is unloaded.

In some embodiments, when the pump 1600 is partially and/or incorrectly loaded with a tubing loop, indicator pin 1616 may be positioned to a height above the top of cap 1680 as shown in FIG. 16B. That is, indicator pin 1616 may be at a height so that at least a portion of it is substantially above cap 1680. This may provide a visual indication to an operator that the pump 1600 is partially and/or incorrectly loaded.

In yet other embodiments, when the pump 1600 is completely and properly loaded with a tubing loop, indicator pin 1616 may be positioned to a height that is substantially flush with the top of cap 1680 as shown in FIG. 16C. That is, indicator pin 1616 may be at a height so that a top of the indicator pin 1616 is at substantially the same height as the top of cap 1680. This may provide a visual indication to an operator that the pump 1600 is completely and properly loaded.

In addition to the height of indicator pin 1616, the side wall of indicator pin 1616 may also have some highlighting feature to provide an additional visual indication. For example, the side wall of pin 1616 may be a different color than the top of pin 1616 and the cap 1680. The different color would aid an operator in identifying when pump 1600 is in a partially and/or incorrectly loaded state, as at least a portion of the sidewall would be visible in this state (see FIG. 16B).

It is noted that by "height" of indicator pin 1616, it is with respect to the cap 1680. That is, in embodiments, the actual height of the indicator pin 1616 may not change, but the cap may move so that the indicator pin 1616 height changes with respect to the cap. In other embodiments, the indicator pin 1616 may actually change heights.

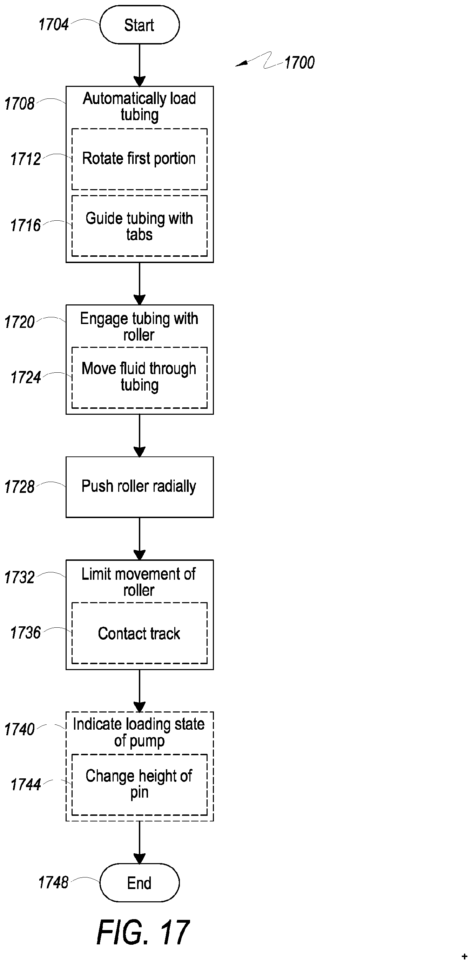

FIG. 17 illustrates a flow chart 1700 that may be performed in embodiments of the present invention for operating a pump. Although specific devices may be described below for performing steps in flow chart 1700, the present invention is not limited thereto. For example, some steps may be described as performed by a first portion or a second portion of a pump. This is done merely for illustrative purposes, and flow chart 1700 is not limited to being performed by any specific device.

Flow chart 1700 illustrates a process consistent with an embodiment of the present invention for operating a pump. In embodiments, flow chart 1700 may be implemented by pumps used in separation systems such as system 100 (FIG. 1). However, in other embodiments, the pumps may be used in different applications.

Flow chart 1700 starts at 1704. Flow passes from 1704 to step 1708, where a tubing loop may be automatically loaded into a pump. In embodiments, the tubing loop may be associated with a disposable set that interfaces with a separation machine (e.g., an apheresis machine). Prior to step 1708, other steps may be performed. For example, an operator may place a tubing loop around a portion of the pump prior to beginning flow 1700 or prior to step 1708.

Step 1708 may include a number of optional sub-steps that are performed as part of step 1708. For example, at optional sub-step 1712, a first portion of a pump (e.g., first portion 304, 1404, 1504, and 1604) may rotate around an axis of rotation. In embodiments, the rotation of the first portion may move the tubing loop into a channel where it is positioned for further operation of the pump.

In some embodiments, optional step 1716 may be performed to guide tubing with tabs into the channel. For example, one or more tabs on a cap of the first portion may be used to help guide the tubing into the channel. As described above with respect to FIGS. 14 and 15, various tab designs, positions, and/or locations, may aid in guiding a tubing loop into a channel of the pump.

After step 1708, flow passes to step 1720, where tubing is engaged by a roller. In embodiments, the rollers may be part of a first portion of the pump. At step 1720, the rollers may engage the tubing, by pressing the tubing against a raceway, which may be part of a second portion of a pump (e.g., second portion 308, 1608). In embodiments, when the pump is being used to move fluid through the tubing, optional step 1724 will be performed as part of step 1720. In other words, by pressing the tubing against the raceway, and the first portion rotating around the axis of rotation, fluid may be moved through the tubing.

In embodiments, the pump may include a spring(s) (e.g., springs 332 and 336) that applies pressure to push the roller radially outward from the axis of rotation. This spring may provide movement to the roller to engage the tubing and press the tubing against the raceway but when the roller is not engaged with the tubing, the roller may move radially outward.

At step 1732, the movement of the roller radially outward may be limited. As discussed above, in some embodiments, when the roller is pushed out radially, it may create a clicking sound that may be amplified by the amount the roller moves. Step 1732 may be performed to limit the movement of the roller after disengaging with the tubing so that the roller does not move as much and therefore does not make a loud clicking sound.

In embodiments, step 1732 may include sub-steps. For example, step 1732 may be performed using a track that limits movement of the roller after it disengages with tubing. The track may be for example similar to track 354 noted above. In a first transition zone, the roller may engage the tubing and not be in contact with the track. As the roller rotates around the raceway, in a second transition zone, the roller may disengage the tubing and contact the track, which limits the radial movement of the roller.

Flow 1700 may also include optional step 1740. The optional step provides an indication of a loading state of the pump. Embodiments provide for providing visual, audio, tactile, indication of whether a pump is unloaded, loaded, partially/incorrectly loaded with tubing. In one embodiment, optional sub-step 1744 may use an indicator pin to perform step 1740. For example, as described above with respect to pump 1600, a height of the indicator pin with respect to a cap, may be used to provide an indication of whether a pump is unloaded, loaded, partially/incorrectly loaded with tubing. Flow 1700 ends at 1748.

Although flow 1700 has been described with steps listed in a particular order, the present invention is not limited thereto. In other embodiments, steps may be performed in different order, in parallel, or any different number of times, e.g., before and after another step. Also, as indicated above, flow 1700 includes some optional steps/sub-steps. However, those steps above that are not indicated as optional should not be considered as essential to the invention, but may be performed in some embodiments of the present invention and not in others.

It will be apparent to those skilled in the art that various modifications and variations can be made to the embodiments of the present invention without departing from its scope. Thus it should be understood that the present invention is not limited to the specific examples provided herein. Rather, the present invention is intended to cover modifications and variations within the scope of the following claims and their equivalents.

While example embodiments and applications of the present invention have been illustrated and described, it is to be understood that the invention is not limited to the precise configuration and applications described above. Various modifications, changes, and variations apparent to those skilled in the art may be made in the arrangement, operation, and details of the features of the present invention disclosed herein without departing from the scope of the present invention.

## Claims

1. A pump (300), the pump comprising:
a first portion (304) that rotates around an axis of rotation (370) when the pump is turned on, the first portion comprising at least one roller (312A) that when tubing is loaded into the pump, the at least one roller engages the tubing; and
a second portion (308) comprising:
a raceway (350), wherein when the tubing is loaded into the pump and the pump is turned on, the at least one roller engages the tubing and the tubing is pressed against the raceway; and
an inner track (354), which is configured such that at least a portion of the inner track comes in contact with the at least one roller when the at least one roller is not engaged with the tubing.

2. The pump of claim 1, wherein the first portion further comprises a spring (332) that presses on the at least one roller to move the at least one roller radially outward.

3. The pump of claim 2, wherein the track limits the amount that the roller may be moved radially outward away from the axis of rotation by the spring.

4. The pump of claim 1, wherein the at least one roller comprises a top edge (320A) and a bottom edge (324A) and at least one of the top edge and the bottom edge is rounded.

5. The pump of claim 1, wherein at least a portion of the inner track is flush with a portion of the raceway.

6. The pump of claim 1, wherein the inner track extends around the entire length of the raceway.

7. The pump of claim 1, wherein the inner track extends around only a portion of a length of the raceway.

8. The pump of claim 1, further comprising a first transition zone (390) and a second transition zone (394).

9. The pump of claim 8, wherein when tubing is loaded into the pump and the pump is turned on, the at least one roller engages the tubing in the first transition zone.

10. The pump of claim 9, wherein when tubing is loaded into the pump and the pump is turned on, the at least one roller disengages the tubing in the second transition zone.

11. The pump of claim 10, wherein when tubing is loaded into the pump and the pump is turned on, the at least one roller contacts the track in the second transition zone.

12. The pump of claim 10, wherein when tubing is loaded into the pump and the pump is turned on, the at least one roller remains in contact with the track until the roller is in the first transition zone.

13. A method of operating a pump (300) comprising:
rotating a first portion (304) of the pump around an axis of rotation (370), the first portion comprising at least one roller (312A);
engaging tubing loaded into the pump with the at least one roller, the tubing being pressed against the raceway (350) of a second portion of the pump (308); and
contacting an inner track (354) of the second portion of the pump with the at least one roller when the at least one roller is not engaged with the tubing.

14. The method of claim 13, wherein the at least one roller engages the tubing in a first transition zone (390).

15. The method of claim 13, wherein the at least one roller disengages the tubing in the second transition zone (394).

## Patentansprüche

1. Pumpe (300), wobei die Pumpe umfasst:
einen ersten Abschnitt (304), der sich um eine Drehachse (370) herum dreht, wenn die Pumpe eingeschaltet ist, wobei der erste Abschnitt wenigstens eine Rolle (312A) umfasst, dass, wenn Rohre in die Pumpe geladen werden, die wenigstens eine Rolle in die Rohre eingreift; und
einen zweiten Abschnitt (308), umfassend:
eine Laufbahn (350), wobei, wenn die Rohe in die Pumpe geladen werden und die Pumpe eingeschaltet ist, die wenigstens eine Rolle in die Rohre eingreift und die Rohre gegen die Laufbahn gedrückt werden; und
eine innere Spur (354), die derart konfiguriert ist, dass wenigstens ein Abschnitt der inneren Spur mit der wenigstens einen Rolle in Berührung kommt, wenn die wenigstens eine Rolle nicht in die Rohre eingreift.

2. Pumpe nach Anspruch 1, wobei der erste Abschnitt ferner eine Feder (332) umfasst, die auf die wenigstens eine Rolle drückt, um die wenigstens eine Rolle radial nach außen zu bewegen.

3. Pumpe nach Anspruch 2, wobei die Spur den Betrag eingrenzt, um den die Rolle durch die Feder radial nach außen von der Drehachse weg bewegt werden kann.

4. Pumpe nach Anspruch 1, wobei die wenigstens eine Rolle eine obere Kante (320A) und eine untere Kante (324A) umfasst und wenigstens eine der oberen Kante und der unteren Kante gerundet ist.

5. Pumpe nach Anspruch 1, wobei wenigstens ein Abschnitt der inneren Spur mit einem Abschnitt der Laufbahn bündig ist.

6. Pumpe nach Anspruch 1, wobei sich die innere Spur um die gesamte Länge der Laufbahn herum erstreckt.

7. Pumpe nach Anspruch 1, wobei sich die innere Spur nur um einen Abschnitt einer Länge der Laufbahn herum erstreckt.

8. Pumpe nach Anspruch 1, ferner umfassend eine erste Übergangszone (390) und eine zweite Übergangszone (394).

9. Pumpe nach Anspruch 8, wobei, wenn die Rohre in die Pumpe geladen werden und die Pumpe eingeschaltet ist, die wenigstens eine Rolle in die Rohre in der ersten Übergangszone eingreift.

10. Pumpe nach Anspruch 9, wobei, wenn die Rohre in die Pumpe geladen werden und die Pumpe eingeschaltet ist, die wenigstens eine Rolle die Rohre in der zweiten Übergangszone freigibt.

11. Pumpe nach Anspruch 10, wobei, wenn die Rohre in die Pumpe geladen werden und die Pumpe eingeschaltet ist, die wenigstens eine Rolle die Spur in der zweiten Übergangszone berührt.

12. Pumpe nach Anspruch 10, wobei, wenn die Rohre in die Pumpe geladen werden und die Pumpe eingeschaltet ist, die wenigstens eine Rolle mit der Spur in Berührung verbleibt, bis sich die Rolle in der ersten Übergangszone befindet.

13. Verfahren zum Betreiben einer Pumpe (300), umfassend:
Drehen eines ersten Abschnitts (304) der Pumpe um eine Drehachse (370) herum, wobei der erste Abschnitt wenigstens eine Rolle (312A) umfasst;
Eingreifen in Rohre, die in die Pumpe geladen werden, mit der wenigstens einen Rolle, wobei die Rohre gegen die Laufbahn (350) eines zweiten Abschnitts der Pumpe (308) gedrückt werden; und
Berühren einer inneren Spur (354) des zweiten Abschnitts der Pumpe mit der wenigstens einen Rolle, wenn die wenigstens eine Rolle nicht in die Rohre eingreift.

14. Verfahren nach Anspruch 13, wobei die wenigstens eine Rolle in die Rohre in der ersten Übergangszone (390) eingreift.

15. Verfahren nach Anspruch 13, wobei die wenigstens eine Rolle die Rohre in der zweiten Übergangszone (394) freigibt.

## Revendications

1. Pompe (300), la pompe comprenant :
une première partie (304) qui entre en rotation autour d'un axe de rotation (370) lorsque la pompe est allumée, la première partie comprenant au moins un rouleau (312A) où, lorsqu'un tuyau est chargé dans la pompe, l'au moins un rouleau entre en prise avec le tuyau ; et
une seconde partie (308) comprenant :
un chemin de roulement (350), dans laquelle, lorsque le tuyau est chargé dans la pompe et la pompe est allumée, l'au moins un rouleau entre en prise avec le tuyau et le tuyau est pressé contre le chemin de roulement ; et
une voie de roulement intérieure (354), qui est configurée de telle sorte qu'au moins une partie de la voie de roulement intérieure entre en contact avec l'au moins un rouleau lorsque l'au moins un rouleau n'est pas en prise avec le tuyau.

2. Pompe selon la revendication 1, dans laquelle la première partie comprend en outre un ressort (332) qui presse sur l'au moins un rouleau pour déplacer l'au moins un rouleau radialement vers l'extérieur.

3. Pompe selon la revendication 2, dans laquelle la voie de roulement limite la quantité selon laquelle le rouleau peut être déplacé radialement vers l'extérieur à l'opposé de l'axe de rotation par le ressort.

4. Pompe selon la revendication 1, dans laquelle l'au moins un rouleau comprend un bord supérieur (320A) et un bord inférieur (324A) et au moins un du bord supérieur et le bord inférieur est arrondi.

5. Pompe selon la revendication 1, dans laquelle au moins une partie de la voie de roulement intérieure est alignée avec une partie du chemin de roulement.

6. Pompe selon la revendication 1, dans laquelle la voie de roulement intérieure s'étend autour de la longueur entière du chemin de roulement.

7. Pompe selon la revendication 1, dans laquelle la voie de roulement intérieure s'étend autour seulement d'une partie d'une longueur du chemin de roulement.

8. Pompe selon la revendication 1, comprenant en outre une première zone de transition (390) et une seconde zone de transition (394).

9. Pompe selon la revendication 8, dans laquelle, lorsqu'un tuyau est chargé dans la pompe et la pompe est allumée, l'au moins un rouleau entre en prise avec le tuyau dans la première zone de transition.

10. Pompe selon la revendication 9, dans laquelle, lorsqu'un tuyau est chargé dans la pompe et la pompe est allumée, l'au moins un rouleau se sépare du tuyau dans la seconde zone de transition.

11. Pompe selon la revendication 10, dans laquelle, lorsqu'un tuyau est chargé dans la pompe et la pompe est allumée, l'au moins un rouleau entre en contact avec la voie de roulement dans la seconde zone de transition.

12. Pompe selon la revendication 10, dans laquelle, lorsqu'un tuyau est chargé dans la pompe et la pompe est allumée, l'au moins un rouleau reste en contact avec la voie de roulement jusqu'à ce que le rouleau soit dans la première zone de transition.

13. Procédé de fonctionnement d'une pompe (300), comprenant :
la mise en rotation d'une première partie (304) de la pompe autour d'un axe de rotation (370), la première partie comprenant au moins un rouleau (312A) ;
la mise en prise d'un tuyau chargé dans la pompe avec l'au moins un rouleau, le tuyau étant pressé contre le chemin de roulement (350) d'une seconde partie de la pompe (308) ; et
la mise en contact d'une voie de roulement intérieure (354) de la seconde partie de la pompe avec l'au moins un rouleau lorsque l'au moins un rouleau n'est pas en prise avec le tuyau.

14. Procédé selon la revendication 13, dans lequel l'au moins un rouleau entre en prise avec le tuyau dans une première zone de transition (390).

15. Procédé selon la revendication 13, dans lequel l'au moins un rouleau se sépare du tuyau dans la seconde zone de transition (394).
